# EUROPÄISCHE PATENTSCHRIFT

(11) **EP 3 244 939 B1**
(45) Veröffentlichungstag und Bekanntmachung des Hinweises auf die Patenterteilung: **09.01.2019**
(21) Anmeldenummer: 16700360.7
(22) Anmeldetag: 12.01.2016
(51) Int. Cl.: A61M 1/10

(54) **BEHÄLTER FÜR EINE HERZPUMPENEINRICHTUNG SOWIE VERFAHREN ZUM BETRIEB EINER HERZPUMPENEINRICHTUNG**
CONTAINER FOR A HEART PUMP DEVICE AND METHOD FOR OPERATING A HEART PUMP DEVICE
RÉCIPIENT POUR UN DISPOSITIF DE POMPE CARDIAQUE ET PROCÉDÉ DE FONCTIONNEMENT D'UN DISPOSITIF DE POMPE CARDIAQUE

(30) Priorität: 13.01.2015 EP 15150897
(43) Veröffentlichungstag der Anmeldung: 22.11.2017
(60) Teilanmeldung: 18200796.3
(73) Patentinhaber: ECP Entwicklungsgesellschaft mbH, 12247 Berlin (DE)
(72) Erfinder: RÖHN, Daniel, 13127 Berlin (DE); SCHUMACHER, Jörg, 14513 Teltow (DE)
(74) Vertreter: Pfenning, Meinig & Partner mbB
(86) Internationale Anmeldenummer: PCT/EP2016/050487
(87) Internationale Veröffentlichungsnummer: WO 2016/113266

(56) Entgegenhaltungen:
- WO-A1-2014/164136

## Beschreibung

Die Erfindung liegt auf dem Gebiet der Mechanik und des Maschinenbaus bzw. der Feinwerktechnik und ist mit besonderem Vorteil im Bereich der Medizintechnik einsetzbar. Insbesondere richtet sich die Erfindung auf eine vorteilhafte Verpackung für eine Herzpumpeneinrichtung.

Zur Unterstützung oder auch zum Ersatz der Herztätigkeit eines Patienten werden in neuerer Zeit zunehmend mechanische Herzpumpen eingesetzt. Grundsätzlich können solche Pumpen innerhalb oder außerhalb des Patientenkörpers betrieben werden. In vielen Fällen ist es jedoch wünschenswert, derartige Pumpen in den Patientenkörper zu implantieren.

In diesem Zusammenhang sind bereits Pumpen bekannt geworden, die sehr stark komprimierbar und expandierbar sind, so dass sie mit geringen Ausmaßen implantiert und im Patientenkörper expandiert werden können. Eine spezielle Art solcher Pumpen weist einen antreibbaren Rotor auf, der in Axialrichtung Blut fördert, wobei der Rotor mit einem Pumpengehäuse vorzugsweise in den Bereich einer Herzkammer bzw. Aorta gebracht und dort betrieben werden kann. Der Transport solcher Pumpen in den Patientenkörper kann beispielsweise durch ein Blutgefäß bis zum Herzen erfolgen.

Selbstverständlich ist bei der Herstellung, dem Transport und der Vorbereitung zur Implantation solcher Herzpumpensysteme eine zuverlässige Sterilität erforderlich. Diese kann gefährdet sein, wenn Teile der Herzpumpeneinrichtung einer sterilen Verpackung nicht fachkundig und sorgfältig entnommen werden. Insbesondere müssen die implantierbaren Teile vor der Berührung mit Gegenständen außerhalb des Patientenkörpers geschützt werden. Beispielsweise kann auch die Kontamination mit Fusseln die Funktion derartiger Pumpen stark beeinträchtigen, und auch die unsachgemäße Berührung mit den Händen könnte unter Umständen eine kritische Beschädigung hervorrufen.

Der vorliegenden Erfindung liegt vor diesem Hintergrund die Aufgabe zugrunde, einen Behälter für eine Herzpumpeneinrichtung zu schaffen, die die Teile der Herzpumpeneinrichtung zuverlässig schützt, steril hält und eine Entnahme mit geringen Kontaminationsrisiken ermöglicht.

Die Aufgabe wird gemäß der Erfindung mit den Merkmalen des unabhängigen Patentanspruchs 1 gelöst. Ausgestaltungen sind in den Unteransprüchen genannt.

Die Erfindung bezieht sich demnach unter anderem konkret auf einen Behälter für eine Herzpumpeneinrichtung mit einem ersten Aufnahmeraum für eine komprimierbare und expandierbare Herzpumpe, wobei der erste Aufnahmeraum auf mehreren Seiten, insbesondere allseitig, von einem oder mehreren Abschlusselementen begrenzt und zur Verhinderung einer Berührung der Herzpumpe nach außen abgeschlossen ist, wobei die Abschlusselemente eine Öffnung zum Durchtritt eines Katheters von außen in den ersten Aufnahmeraum freilassen, wobei der Durchmesser der Öffnung derart bemessen ist, dass die Herzpumpe diese ausschließlich in einem gegenüber dem expandierten Zustand wenigstens teilweise komprimierten Zustand passieren kann. Eine Herzpumpeneinrichtung umfasst demnach wenigstens eine komprimierbare und expandierbare Herzpumpe sowie gegebenenfalls weitere Teile, wie beispielsweise einen Katheter und/oder eine Katheterspüleinrichtung und eine innerhalb des Katheters verlaufende Antriebswelle für die Herzpumpe, und gegebenenfalls noch weitere Teile.

Für eine derartige Herzpumpeneinrichtung soll ein Behälter bereitgestellt werden. Dieser weist einen ersten Aufnahmeraum auf, in dem die Herzpumpe derart untergebracht werden kann, dass sie durch die Abschlusselemente vor Berührungen, beispielsweise durch Bedienpersonal, geschützt ist. Dabei können die Abschlusselemente den Abschlussraum mit Ausnahme der Öffnung zum Durchtritt eines Katheters fluiddicht abschließen; es kann jedoch auch vorgesehen sein, dass die Abschlusselemente Öffnungen aufweisen, dass beispielsweise eines der Abschlusselemente gitterartig aufgebaut ist. Hierdurch kann z. B. eine optische Kontrolle der in dem ersten Aufnahmeraum befindlichen Herzpumpe ermöglicht sein. Es kann jedoch auch wenigstens ein Teil der Abschlusselemente wenigstens teilweise optisch transparent ausgestaltet sein, um einen Einblick in den ersten Aufnahmeraum zu ermöglichen.

Die Öffnung zum Durchtritt eines Katheters dient dazu, einen mit einer Herzpumpe unmittelbar verbundenen Katheter durchzuführen, wobei vorteilhaft die gesamte Länge des Katheters außerhalb des ersten Aufnahmeraums in einem zweiten Aufnahmeraum des Behälters Platz findet. Schließlich kann auch ein Griff, der an dem Katheter zur Handhabung angeordnet ist, in einem dritten Aufnahmeraum des Behälters Platz finden und dort beispielsweise ebenfalls allseitig von Wänden des Behälters umgeben sein.

Der Durchmesser der Öffnung soll derart bemessen sein, dass die Pumpe die Öffnung bei Entnahme (beim Verpacken könnte die Öffnung zunächst größer sein, die erst danach durch einen Deckel verschlossen würde) nicht in expandiertem Zustand passieren kann. Beispielsweise kann vorgesehen sein, dass die Pumpe in expandiertem Zustand in dem ersten Aufnahmeraum gelagert wird, um dort auch testweise in Betrieb genommen werden zu können. Ein Rotor der Pumpe kann beispielsweise mittels einer durch den Katheter verlaufenden Antriebswelle in Rotation versetzt werden, um die Betriebsbereitschaft der Herzpumpe zu überprüfen. Die begrenzte Größe der Öffnung stellt sicher, dass die Herzpumpe in diesem expandierten Zustand nicht einfach aus dem ersten Aufnahmeraum entfernt werden, beispielsweise mittels des Katheters herausgezogen werden kann. Entscheidend ist hier eher, dass die Pumpe bzw. der Pumpenkopf nicht mit der Hand berührt werden kann. Damit wird das Risiko einer unbedachten Berührung der Herzpumpe weiter reduziert. Durch die Bemessung der Öffnung wird sichergestellt, dass bei einem Herausziehen der Herzpumpe aus dem ersten Aufnahmeraum diese wenigstens teilweise komprimiert wird, so dass sie unmittelbar in eine an der Außenseite des ersten Aufnahmeraums im Bereich der Öffnung angesetzte Schleuse hineingezogen werden kann, die die Pumpe in wenigstens teilweise komprimiertem Zustand aufnimmt. Es kann vorgesehen sein, dass der Durchmesser der Öffnung kleiner als 6 mm, vorzugsweise kleiner als 5 mm, besonders vorzugsweise kleiner als 4 mm ist.

Eine vorteilhafte Ausgestaltung des erfindungsgemäßen Behälters sieht vor, dass wenigstens die Abschlusselemente, insbesondere der gesamte Behälter, im Wesentlichen aus einem Kunststoffflachmaterial, insbesondere einer Kunststofffolie, bestehen. Der Behälter kann beispielsweise als sogenannter Blister aus einer biegesteifen Kunststofffolie hergestellt sein. Es kann jedoch auch ein stärkeres Kunststoffmaterial vorgesehen sein, oder Teile des Behälters können aus Kunststoffelementen bestehen, die in einem Spritzgießverfahren hergestellt sind. Besteht der Behälter im Wesentlichen aus einem Blister, so kann er aus einer Kunststofffolie im Tiefziehverfahren oder im Pressverfahren geformt werden.

Die Erfindung sieht vor, dass der erste Aufnahmeraum im Wesentlichen durch zwei halbschalenförmige, zusammengefügte Abschlusselemente begrenzt ist. Der Aufnahmeraum kann beispielsweise eine erste Halbschale in Form einer Mulde zur Aufnahme der Herzpumpe aufweisen, die den ersten Aufnahmeraum teilweise begrenzt, und es kann wenigstens ein weiteres Abschlusselement als Halbschale auf die erste Halbschale aufgesetzt werden, um den ersten Aufnahmeraum vollständig abzuschließen. Die zweite Halbschale, die somit einen Deckel bildet, kann mit der ersten Halbschale durch Verpressen, Verkleben, Verschweißen oder sonstige Fügeverfahren verbunden sein und ist vorteilhaft nicht zerstörungsfrei ablösbar. Dadurch kann sichergestellt werden, dass eine Herzpumpe, die herstellerseitig im Aufnahmeraum angeordnet wird und die vor ihrem Einsatz bei einer Implantation sich immer noch in diesem ersten Aufnahmeraum befindet, in der Zwischenzeit nicht aus diesem entfernt worden sein kann.

Die Erfindung kann somit vorsehen, dass die Abschlusselemente unlösbar oder schwer lösbar miteinander verbunden sind.

Die beiden Halbschalen können mit einander zugewandten Öffnungen oder mit übereinstimmender Ausrichtung der Öffnungen aufeinandergesetzt sein.

Es kann zudem vorteilhaft vorgesehen sein, dass die Öffnung zum Durchtritt eines Katheters zwischen zwei Abschlusselementen gebildet oder zur Fügestelle zwischen zwei Abschlusselementen hin offen ist. Damit ist das Einlegen der Herzpumpe und des mit dieser verbundenen Katheters in eine Öffnung vor dem Zusammenfügen mehrerer Abschlusselemente zur Begrenzung des ersten Aufnahmeraums besonders einfach möglich.

Wie oben erwähnt, kann vorteilhaft vorgesehen sein, dass ein erstes der Abschlusselemente als Teil eines Blisters ausgebildet ist, der wenigstens einen Katheter, insbesondere zusätzlich weitere Teile einer Herzpumpeneinrichtung aufnimmt.

Es kann dabei zudem vorgesehen sein, dass das erste Abschlusselement im Bereich des ersten Aufnahmeraums eine Auffangschale für Flüssigkeit bildet. Damit wird erleichtert, dass die Herzpumpe in dem ersten Aufnahmeraum für einen Testbetrieb mit einer Flüssigkeit benetzt werden kann. Die Benetzung ist darüber hinaus auch für die beschädigungsfreie Komprimierung der Pumpe vorteilhaft. Die Flüssigkeit kann beispielsweise über den Katheter der Herzpumpe zugeführt werden. Die Auffangschale sorgt dafür, dass die Flüssigkeit sich einerseits um die Pumpe herum sammelt und damit die Benetzung der Herzpumpe sicherstellt und andererseits nicht aus dem ersten Aufnahmeraum in unkontrollierter Weise entweicht.

Die Erfindung kann zudem vorteilhaft dadurch ausgestaltet werden, dass die Öffnung wenigstens teilweise aus einem zylindersymmetrischen Kanal besteht. Ein derartiger zylindersymmetrischer Kanal mit vorzugsweise glatten Wänden ermöglicht das Durchziehen einer Herzpumpe bei gleichzeitiger Kompression, ohne das Gehäuse der Herzpumpe zu verletzen. Vorzugsweise kann dabei vorgesehen sein, dass der zylindersymmetrische Kanal sich vom Inneren des ersten Aufnahmeraums aus nach außen verengt und im Bereich, den die Pumpe passiert, keine scharfen Kanten aufweist.

Der zylindersymmetrische Kanal kann zu diesem Zweck beispielsweise konisch oder abschnittsweise konisch ausgebildet sein. Der Kanal kann beispielsweise an seiner Mündung zum Inneren des ersten Aufnahmeraums einen Einführtrichter aufweisen, in den die Herzpumpe unter wenigstens teilweiser Kompression hineingezogen werden kann.

Zudem kann vorteilhaft vorgesehen sein, dass die Mündung der Öffnung zur Außenseite der Abschlusselemente und des ersten Aufnahmeraums einen Rand aufweist, an dem ein längs des Katheters verschiebbares hohlzylindrisches Schleusenelement in Axialrichtung des Kanals abstützbar ist.

Eine Schleuse in Form eines hohlzylindrischen Bauteils, insbesondere beispielsweise eine Peel-away-Schleuse, die durch Zerstörung beim Aufreißen radial entfernt werden kann, kann über den Katheter gezogen bzw. dieser in die Schleuse hineingezogen werden und ist üblicherweise bereits Bestandteil der in dem Behälter befindlichen Herzpumpeneinrichtung. Ein solches Schleusenelement kann von der Außenseite des ersten Aufnahmeraums her gegen den Rand der Öffnung an den Abschlusselementen gedrückt werden, so dass die Herzpumpe mittels des Katheters, der durch das Schleusenelement hindurch verläuft, aus dem ersten Aufnahmeraum durch die Öffnung heraus- und in das Schleusenelement hineingezogen werden kann. Dabei wird die Herzpumpe bereits innerhalb der Öffnung des Aufnahmeraums wenigstens teilweise radial komprimiert und insbesondere beim Eintritt in das Schleusenelement noch weiter radial komprimiert. Es kann jedoch auch vorgesehen sein, dass der Innendurchmesser des Schleusenelements dem Durchmesser entspricht, auf den die Herzpumpe bereits in der Öffnung des Aufnahmeraums komprimiert ist, so dass eine weitere Komprimierung beim Einziehen in die Schleuse entfällt. Der Rand der Öffnung kann vorteilhaft eine zur Längsachse der Öffnung senkrecht verlaufende ringförmige Fläche aufweisen.

Ist die Herzpumpe in das Schleusenelement eingezogen, so kann die Herzpumpeneinrichtung dem Behälter entnommen und mittels einer Einführschleuse in einen Patientenkörper eingeführt werden. Dazu wird das Schleusenelement an die Einführschleuse angekoppelt, und die Herzpumpe wird aus dem Schleusenelement heraus in die Einführschleuse hinein verschoben. Ist das Schleusenelement als Peel-away-Schleuse ausgebildet, so kann diese problemlos nach dem Einführen der Herzpumpe in die Einführschleuse entfernt werden. Es kann aber auch das Schleusenelement selbst als Einführschleuse verwendet werden, indem man das Schleusenelement mit der darin komprimierten Pumpe, beispielsweise über einen Führungsdraht, in den Patienten einführt. Hierfür ist gegebenenfalls ein separates Lumen für den Führungsdraht vorzusehen.

Die Erfindung bezieht sich außer auf einen Behälter der oben beschriebenen Art auch auf einen Behälter in entsprechender Ausführung mit einer Herzpumpeneinrichtung, wobei eine komprimierbare und expandierbare Herzpumpe sich im ersten Aufnahmeraum befindet und wobei ein mit der Herzpumpe verbundener Katheter durch die Öffnung aus dem ersten Aufnahmeraum herausragt, wobei insbesondere auf dem Katheter frei verschiebbar ein von diesem durchsetztes Schleusenelement vorgesehen ist.

Die Erfindung bezieht sich zudem auf ein Verfahren zum Betrieb einer Herzpumpeneinrichtung, bei dem eine Herzpumpe im ersten Aufnahmeraum eines Behälters nach einem der Ansprüche 1 bis 12 angeordnet ist und beispielsweise mittels einer durch einen Katheter zur Herzpumpe verlaufenden Welle von außen rotatorisch angetrieben wird (eine Pumpe mit integriertem Motor am Pumpenkopf ist jedoch auch möglich). Dieses Verfahren erlaubt es, die Funktionsfähigkeit einer mit einem Rotor versehenen Herzpumpe bereits im Behälter zu überprüfen, ohne dass die Gefahr einer Berührung und Desterilisation besteht. Der Testbetrieb findet üblicherweise bei Drehzahlen unterhalb der Betriebsdrehzahl im Patientenkörper statt, beispielsweise bei 50 %, insbesondere auch nur bei höchstens 30 % oder 10 % der Drehzahl.

Es kann vorteilhaft vorgesehen sein, dass hierzu durch eine an dem Katheter vorgesehene Öffnung entlang dem Katheter eine Flüssigkeit zur Herzpumpe gefördert wird. Damit wird sichergestellt, dass die Herzpumpe unter realistischen Bedingungen in Berührung mit einer Flüssigkeit getestet wird. Gleichzeitig kann die Herzpumpeneinrichtung so weit wie möglich mit der Flüssigkeit gefüllt werden, um Lufteinschlüsse bei der Implantation weitgehend zu vermeiden. Als Flüssigkeit, die zur Herzpumpe gefördert wird, kommt jede übliche bioverträgliche Spülflüssigkeit, beispielsweise Kochsalzlösung, Glucoselösung oder Ähnliches, in Frage.

Die Erfindung bezieht sich zudem auf ein Verfahren zum Betrieb einer Herzpumpeneinrichtung, bei dem eine Herzpumpe im ersten Aufnahmeraum eines Behälters nach einem der Ansprüche 1 bis 12 angeordnet ist, wobei die Herzpumpe mit einem Katheter verbunden ist, der durch die Öffnung aus dem ersten Aufnahmeraum herausragt, dadurch gekennzeichnet, dass mittels des Katheters die Herzpumpe durch die Öffnung unter radialer Kompression aus dem ersten Aufnahmeraum heraus- und in ein Schleusenelement, das auf dem Katheter in Axialrichtung (das heißt in Längsrichtung des Katheters) frei verschiebbar ist, hineingezogen wird.

Die in den unabhängigen Ansprüchen aufgeführten Gegenstände beinhalten unter anderem, dass ein oder mehrere Abschlusselemente einen Aufnahmeraum für eine komprimierbare und expandierbare Herzpumpe begrenzen. Unter "Aufnahmeraum" wird vorzugsweise der kleinste zusammenhängende Raum verstanden, der den Pumpenkopf einer Herzpumpe vollständig umschließt (hierzu sollen nicht, beispielsweise an eine Entnahmeöffnung 7 (siehe Figuren, unten) sich anschließende weitere Bauteile gehören). Dies erfolgt zur Verhinderung einer Berührung der Herzpumpe, d.h., dass die Herzpumpe im Aufnahmeraum vor Berührung geschützt ist. Die Abschlusselemente lassen zumindest eine Öffnung zum Durchtritt eines Katheters frei, wobei der Durchmesser der Öffnung derart bemessen ist, dass die Herzpumpe diese ausschließlich in einem gegenüber dem expandierten Zustand wenigstens teilweise komprimierten Zustand passieren kann. Hierbei ist mit "zum Durchtritt eines Katheters" gemeint, dass ein entsprechender Katheter diese Öffnung passiert, hiermit ist vorzugsweise nicht eine bestimmte Durchtrittsrichtung gemeint. In den unabhängigen Ansprüchen ist von "einem oder mehreren Abschlusselementen" die Rede. Insbesondere bei dem Fall von "mehreren Abschlusselementen" können dies unterschiedliche Gestaltungen sein. Das heißt, die Abschlusselemente, die den Aufnahmeraum bilden, können auf verschiedene Weise miteinander gefügt sein. So sind Verbindungen beispielsweise als Schraubverbindungen etc. realisierbar. Vorzugsweise ist die Fügung derart, dass diese nicht zerstörungsfrei lösbar ist, allerdings alternativ auch zerstörungsfrei herstellbar. Somit kann beispielsweise eine expandierte Pumpe in dem Aufnahmeraum untergebracht werden, indem diese Abschlusselemente "um die Pumpe herum" zusammengefügt werden und danach nicht mehr zerstörungsfrei voneinander getrennt werden können. In diesem Sinne kann eine Fügelinie, die unterschiedliche Abschlusselemente miteinander verbindet, entweder in Längsrichtung eines Katheters, der aus einer Öffnung des Aufnahmeraums ragt, angeordnet sein, oder auch orthogonal zu dieser Richtung. Im Folgenden werden hierzu weitere Details angegeben.

In einer Ausführungsform ist der erste Aufnahmeraum durch mindestens zwei entlang einer Fügelinie gefügte Abschlusselemente begrenzt, wobei die Fügelinie in einem Querschnitt des Aufnahmeraums verläuft, der größer ist als der Querschnitt der Öffnung zum Durchtritt eines Katheters. Auf diese Weise ist es möglich, dass die Abschlusselemente "um den Pumpenkopf herum" gefügt werden, ein späteres Herausziehen der Herzpumpe (des Pumpenkopfes) ist nur unter Kompression möglich.

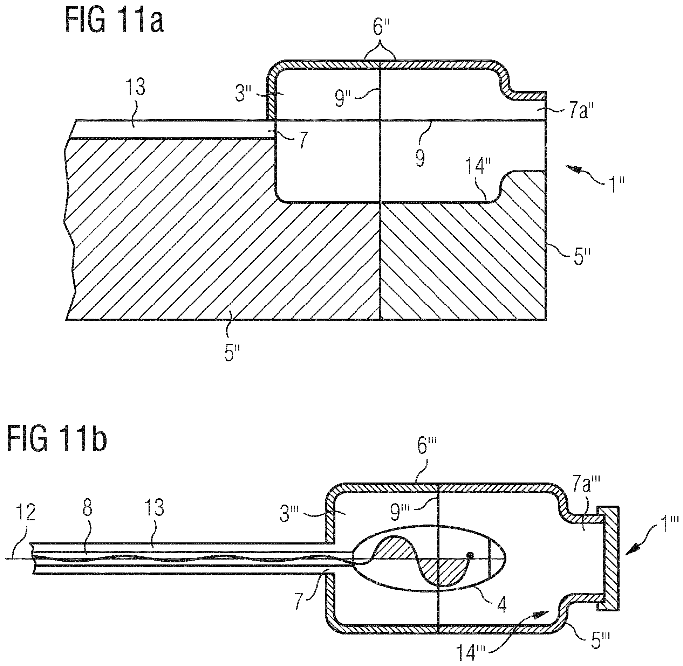

Eine weitere Ausbildung sieht vor, dass der Aufnahmeraum mehr als eine Öffnung aufweist. Hierzu ist es beispielsweise möglich, dass die komprimierbare und expandierbare Herzpumpe so in den Aufnahmeraum hineingezogen wird, dass das Hineinbringen der expandierbaren Herzpumpe in den Aufnahmeraum im Wesentlichen ohne Kompression erfolgen kann. Allerdings ist die Herausnahme des Pumpenkopfes (vorzugsweise am gegenüberliegenden Ende des Aufnahmeraums) dann nur unter Kompression möglich (siehe beispielsweise Fig. 11a).

Eine Weiterbildung sieht vor, dass mindestens eine der Öffnungen des Aufnahmeraums durch einen zerstörungsfrei oder auch nicht zerstörungsfrei entfernbar an diese Öffnung des Aufnahmeraums gefügten Deckel verschlossen ist. So ist es beispielsweise möglich, dass auf die oben beschriebene Weise eine Pumpe zunächst durch die größere Öffnung (die nicht zwingend eine Kompression der Herzpumpe erzwingt) in den Aufnahmeraum hineingezogen wird. Anschließend ist es möglich, diesen durch einen Deckel derart zu verschließen, dass der Deckel nicht zerstörungsfrei lösbar ist. Auf diese Weise wird sichergestellt, dass die Herzpumpe nicht durch einen Benutzer ohne Kompression aus dem Aufnahmeraum entnehmbar ist.

Es ist also möglich, den Aufnahmeraum so mit Öffnungen zu versehen, dass die Herzpumpeneinrichtung/die Herzpumpe/der Pumpenkopf ohne Kompression in den Aufnahmeraum einführbar sind, allerdings ausschließlich in einem gegenüber dem expandierten Zustand wenigstens teilweise komprimierten Zustand entnehmbar ist.

Im Folgenden wird die Erfindung anhand eines Ausführungsbeispiels in Figuren einer Zeichnung gezeigt und nachfolgend beschrieben. Dabei zeigt
- Fig. 1: in einem Querschnitt schematisch einen erfindungsgemäßen Behälter,
- Fig. 2: im Querschnitt einen erfindungsgemäßen Behälter mit einer Herzpumpe,
- Fig. 3: einen Behälter in einem weiteren Querschnitt,
- Fig. 4: eine Herzpumpe mit einem Katheter,
- Fig. 5: eine Draufsicht auf einen Behälter mit einer Herzpumpe,
- Fig. 6: einen Querschnitt durch einen Behälter mit einer Herzpumpe und einem Schleusenelement,
- Fig. 7: einen anderen Querschnitt durch einen Behälter sowie ein Schleusenelement,
- Fig. 8: eine dreidimensionale Ansicht eines Behälters in der Ausführungsform als Blister,
- Fig. 9: einen Querschnitt durch einen als Blister ausgeführten Behälter,
- Fig. 10: einen Ausschnitt aus einem als Blister ausgeführten Behälter in dreidimensionaler Ansicht sowie
- Fign. 11a und 11b: Querschnitte bzw. Teilquerschnitte weiterer Ausführungsformen eines erfindungsgemäßen Behälters.

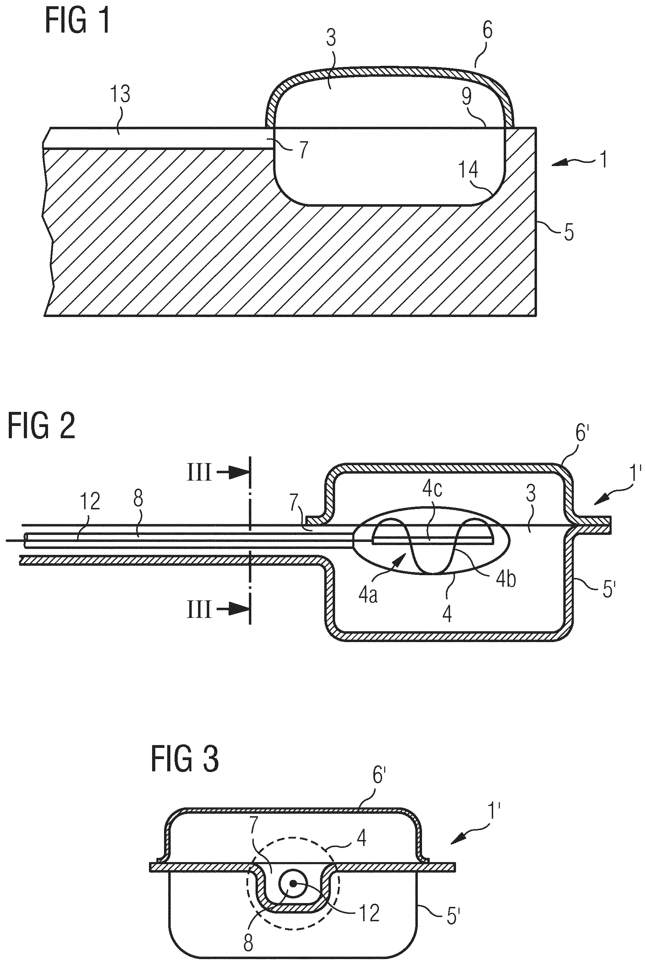

Figur 1 zeigt in einem Querschnitt einen Behälter 1 mit zwei Abschlusselementen 5, 6, wobei das erste Abschlusselement 5 als massiver Körper ausgebildet ist, während das zweite Abschlusselement 6 als dünnwandige Halbschale in Form eines Deckels den ersten Aufnahmeraum 3 abschließt. Es ist eine Öffnung 7 im Bereich der Abschlusselemente 5, 6, und zwar speziell als Ausnehmung in dem ersten Abschlusselement 5, vorgesehen, durch die ein Katheter aus dem ersten Aufnahmeraum 3 in den Außenraum austreten kann. Für die Aufnahme des Katheters ist eine Rinne 13 vorgesehen, die im weiteren Verlauf des Behälters 1 ringförmig verlaufen kann, um die Ablage einer oder mehrerer Schleifen eines Katheters zu ermöglichen. Die massive Darstellung des ersten Abschlusselements 5 ist hier nur beispielhaft gegeben, um die grundsätzliche Funktion zu erläutern.

Das erste Abschlusselement 5 sollte eine fluiddichte Auffangwanne 14 bilden, in die eine Herzpumpe eingelegt werden kann und die bei einem Probebetrieb Flüssigkeit auffangen kann.

Das zweite Abschlusselement 6 ist vorzugsweise mit dem ersten Abschlusselement 5 unlösbar und dicht im Bereich der Fügestelle 9 verbunden, wobei die Verbindung mit Ausnahme der Öffnung 7 vorteilhaft auch fluiddicht, aber nicht luftdicht (da die Luft aus dem Katheter hier entweichen soll), ausgeführt sein kann. Die Fügestelle 9 bildet bei diesem Beispiel eine Fügelinie bzw. eine ringförmige Fügefläche, die insgesamt hier in einer Ebene liegt.

Das zweite Abschlusselement 6 kann fluiddicht als gebogenes, flaches Kunststoffteil, beispielsweise als steife Folie, ausgebildet sein, es kann jedoch auch Öffnungen und/oder ein oder mehrere optische Fenster aufweisen, um den Einblick in den ersten Aufnahmeraum 3 zu ermöglichen. Entscheidend für das zweite Abschlusselement 6 ist, dass es die in dem ersten Aufnahmeraum 3 aufzubewahrende Herzpumpe vor Berührungen schützt.

In Figur 2 ist ein Behälter 1' gezeigt, bei dem das erste Abschlusselement 5' ebenso wie das zweite Abschlusselement 6' als steife Folie in Form eines Blisters ausgebildet ist. Der erste Aufnahmeraum 3 ist ebenso wie bei dem Ausführungsbeispiel der Figur 1 gebildet, und in Figur 2 ist zudem eine im ersten Aufnahmeraum 3 angeordnete Herzpumpe 4 schematisch dargestellt. Die Herzpumpe 4 weist einen Rotor 4a mit einem wendeiförmigen Förderelement 4b und einer Nabe 4c auf, wobei die Nabe 4c mit einer antreibbaren flexiblen Antriebswelle 12 verbunden ist. Die Antriebswelle 12 verläuft aus der Herzpumpe 4 heraus durch einen Katheter 8, der die Öffnung 7 passiert.

Die Herzpumpe 4 ist im nichtkomprimierten Zustand dargestellt, in dem ihre radiale Ausdehnung senkrecht zu der durch die Nabe 4c angedeuteten Axialrichtung größer ist als die Ausdehnung der Öffnung 7.

In Figur 3 ist ein Schnitt, der in Figur 2 bereits mit III bezeichnet und angedeutet ist, mit einem Blick auf das erste Abschlusselement 5' und das zweite Abschlusselement 6' sowie die Öffnung 7 dargestellt, wobei der Katheter 8 mit der Antriebswelle 12 eingezeichnet ist und wobei in gestrichelter Form der Umriss der Herzpumpe 4 eingezeichnet ist. Aus Figur 3 ist ersichtlich, dass der Durchmesser der Herzpumpe 4 größer ist als die lichte Weite der Öffnung 7, so dass die Herzpumpe an dem Katheter 8 nur unter gleichzeitiger radialer Kompression durch die Öffnung 7 aus dem ersten Aufnahmeraum 3 herausziehbar ist.

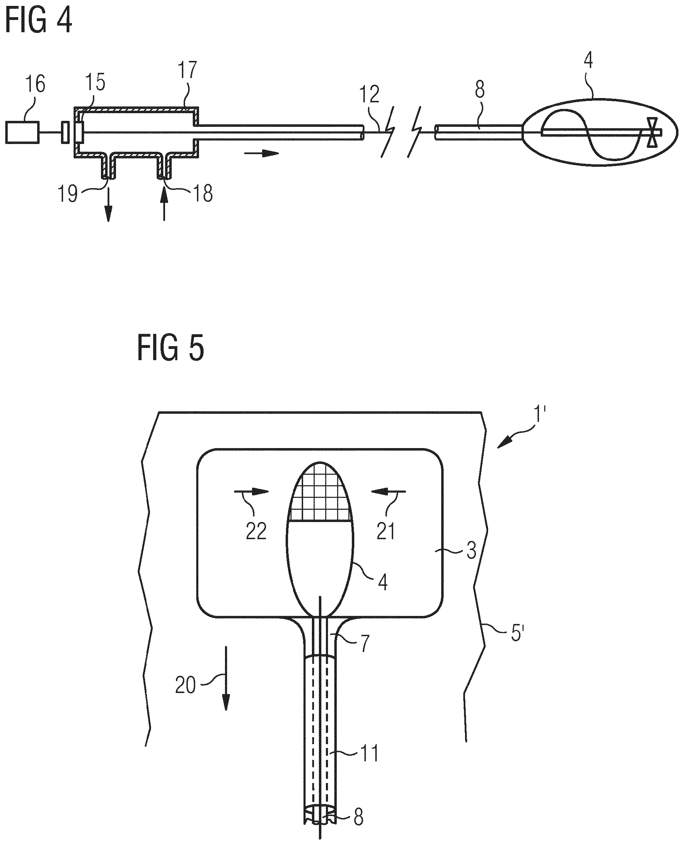

Figur 4 zeigt eine Herzpumpeneinrichtung mit einer Herzpumpe 4, die anhand der Figur 2 bereits beschrieben wurde, sowie mit einem Katheter 8 und einer Antriebswelle 12, wobei zudem noch das antriebsseitige Ende der Antriebswelle 12 mit einer Magnetkupplung 15 gezeigt ist, die die Übertragung einer Antriebsbewegung von einem Motor 16 zum Inneren eines Behälters 17 erlaubt, in dem die Antriebswelle 12 an die Magnetkupplung angekoppelt ist.

Der Behälter 17 dient zudem als Spüleinrichtung mit mehreren Spülöffnungen 18, 19, wobei durch die Öffnung 18 eine Spülflüssigkeit, beispielsweise Kochsalzlösung, in den Behälter 17 eingeführt und überschüssige Spülflüssigkeit durch die zweite Spülöffnung 19 entfernt wird. Die Spülflüssigkeit bewegt sich zudem entlang des Katheters 8 in Richtung auf die Pumpe 4 und wird insbesondere beim Betrieb des Rotors, d. h. bei einer Drehung der Antriebswelle 12, durch eine wendelförmige Außenkontur der Antriebswelle 12 in Richtung zur Pumpe 4 hin gefördert. Für einen Probebetrieb kann somit Spülflüssigkeit durch die erste Spülöffnung 18 zugeführt und durch den Katheter 8 zur Pumpe 4 bewegt werden, während diese sich im ersten Aufnahmeraum 3 befindet, und sodann kann die Pumpe zumindest bei reduzierter Drehzahl unter Benetzung durch die Spülflüssigkeit probeweise betrieben werden.

Figur 5 zeigt in einer Draufsicht von oben ein erstes Abschlusselement 5' sowie das Innere des ersten Aufnahmeraums 3, in dem eine Herzpumpe 4 angeordnet ist. Der mit der Herzpumpe 4 verbundene Katheter 8 ragt durch die Öffnung 7 hindurch und ist außerhalb des Aufnahmeraums 3 vor der Öffnung 7 von einem Schleusenelement 11 umgeben. Das Schleusenelement 11 ist beispielsweise als flexibler Kunststofftorus in Form eines Schlauchabschnitts ausgebildet, der eine Sollbruchstelle aufweisen kann, so dass er als Peel-away-Schleuse später nach dem Überführen der Pumpe 4 in eine Einführschleuse am Patientenkörper radial weggezogen werden kann.

Das Schleusenelement 11 kann auf den Rand der Öffnung 7 an den Abschlusselementen 5', 6' des ersten Aufnahmeraums 3 von außen angesetzt werden, und darauf kann die Pumpe 4 mittels des Katheters in Richtung des Pfeils 20 aus dem Aufnahmeraum 3 herausgezogen werden. Die Pumpe 4 wird aufgrund des gegebenen Durchmessers der Öffnung 7, der kleiner ist als der Pumpendurchmesser im expandierten Zustand, beim Einziehen in die Öffnung 7 in Richtung der Pfeile 21, 22 radial komprimiert und im komprimierten oder wenigstens teilweise komprimierten Zustand in das Schleusenelement 11 eingezogen. Dort ist es wieder vor Berührung und Kontamination geschützt und kann dem Behälter 1' entnommen und zu einer Einführschleuse am Körper eines Patienten bewegt werden.

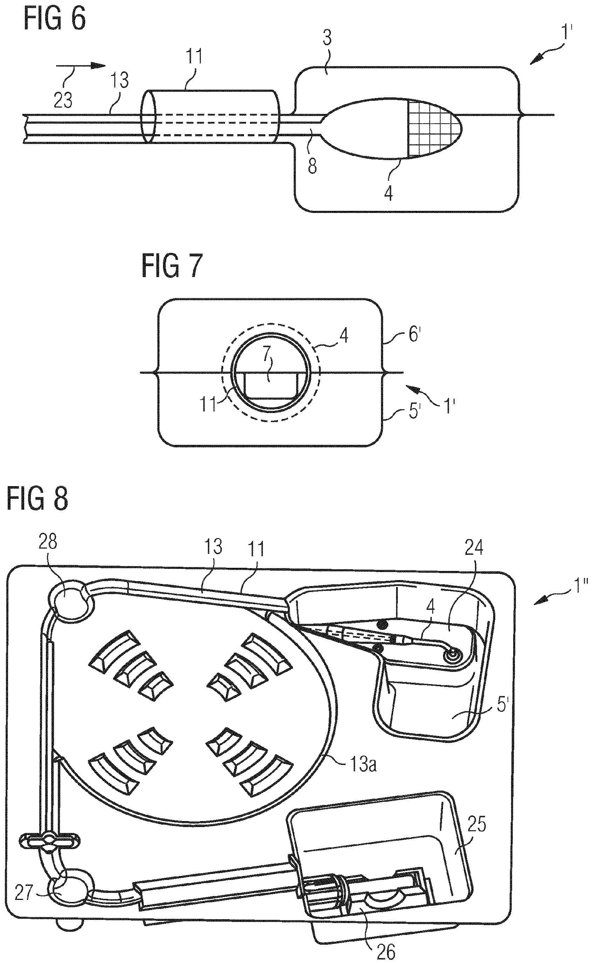

Figur 6 zeigt nochmals in einer Seitenansicht schematisch einen Behälter 1' mit einem ersten Aufnahmeraum 3, in dem eine Pumpe 4 angeordnet ist, sowie ein in einer Rinne 13 des Behälters 1' angeordnetes Schleusenelement 11, das einen Katheter 8 umgibt.

Figur 7 zeigt in einer weiteren Ansicht in Richtung des Pfeils 23 in Figur 6 gesehen eine äußere Ansicht der Abschlusselemente 5', 6' mit der Öffnung 7 und einer Draufsicht auf das Schleusenelement 11 in axialer Richtung sowie in gestrichelter Darstellung die im ersten Aufnahmeraum 3 liegende Herzpumpe 4.

Figur 8 zeigt in einer perspektivischen Ansicht ein erstes Abschlusselement 5', das als Teil eines Blisters ausgeführt ist, mit zwei Mulden, von denen eine erste 24 durch das erste Abschlusselement 5' begrenzt ist und den unteren Teil eines ersten Aufnahmeraums für eine Herzpumpe bildet, während die zweite Mulde 25 einen dritten Aufnahmeraum für einen Griff 26 an dem Katheter 8 begrenzt. Es ist zudem eine Rinne 13 erkennbar, die von der ersten Mulde 24 zur zweiten Mulde 25 führt, einen zweiten Aufnahmeraum bildet und die Ablage eines Katheters erlaubt, wobei zudem eine bogenförmige Zusatzrinne 13a gebildet ist, die die Ablage von Schleifen des Katheters erlaubt. Zudem sind Griffmulden 27, 28 im Bereich der Rinne 13 vorgesehen, die einerseits zur Stabilisierung des Blisters und andererseits zum besseren Greifen eines in der Rinne 13 befindlichen Katheters bei der Entnahme dienen, sowie zudem zur Ausbildung von Ausstülpungen des Blisters, die als Abstützelemente beim Abstellen auf einer ebenen Fläche neben den Mulden 24, 25 dienen können.

Innerhalb des Behälters 1" bzw. des Abschlusselements 5' sind eine Herzpumpe 4 sowie das Griffteil 26 dargestellt. Üblicherweise ist jedoch zur Vervollständigung des Behälters 1" jeweils ein Abschlusselement sowohl auf der ersten Mulde 24 als auch auf der zweiten Mulde 25 vorgesehen, um die jeweiligen Mulden und die darin befindlichen Bauteile abzudecken und vor Berührung zu schützen sowie um die Komponenten erschütterungssicher zu fixieren. Aus diesem Grund ist auch die atraumatische Spitze des Katheters (sogenannte Pigtailspitze) so fixiert, dass sie übermäßige Bewegungen des Pumpenkopfes nicht zulässt, andererseits aber das Herausziehen in Richtung des Schleusenelements 11 nicht behindert wird.

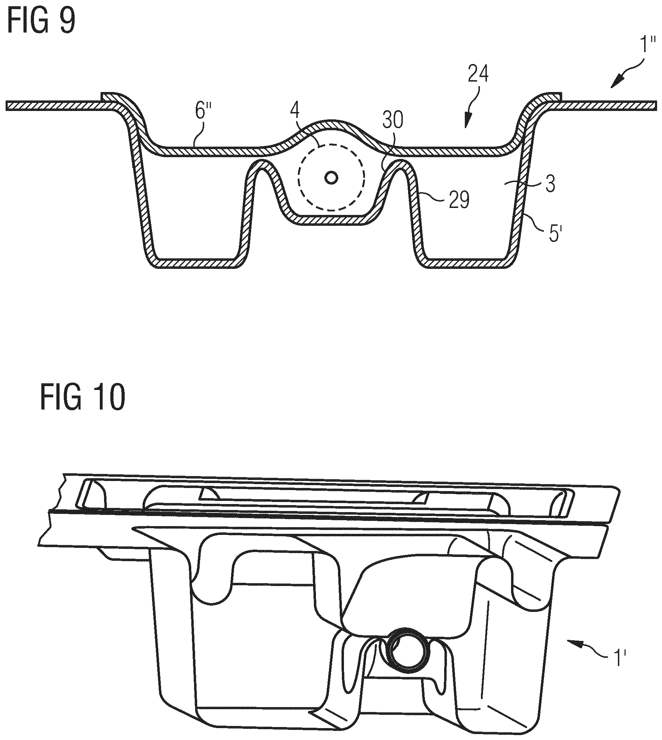

In Figur 9 ist beispielhaft ein Querschnitt durch die Mulde 24 gezeigt, die in dem als Teil eines Blisters ausgebildeten ersten Abschlusselement 5' gebildet ist. Innerhalb des Mulde und innerhalb des ersten Aufnahmeraums 3 ist eine Erhebung 29 durch eine entsprechende Hochwölbung des ersten Abschlusselements 5' gebildet, wobei die Erhebung 29 in sich eine Vertiefung 30 aufweist, die innerhalb des ersten Aufnahmeraums 3 die Herzpumpe 4 aufnimmt. Damit ist eine genaue, enge und zwingende Positionierung der Herzpumpe 4 in dem ersten Aufnahmeraum 3 gegeben. Der erste Aufnahmeraum 3 ist zudem mit einem zweiten Abschlusselement 6" in Form einer steifen Kunststofffolie vollständig abgedeckt, wobei das zweite Abschlusselement 6" im Bereich der Berührungsfläche mit dem ersten Abschlusselement 5' mit diesem verklebt, verschweißt oder verpresst (beispielsweise auch durch druckknopfähnliche Verbindungen) sein kann, so dass eine Ablösung des zweiten Abschlusselements 6" ohne eine Zerstörung des Behälters 1" nicht oder nur sehr schwer möglich ist. Die offenen Seiten des ersten und zweiten Abschlusselements 5', 6" sind in diesem Beispiel gleichsinnig ausgerichtet und nicht, wie es grundsätzlich auch möglich wäre, einander zugewandt.

Die Deckelform des die zweite Mulde 25 abdeckenden Abschlusselements kann ähnlich der Form des zweiten Abschlusselements 6" gestaltet sein.

Bei der Vorbereitung einer Implantation einer Herzpumpe, wie sie in Figur 8 dargestellt ist, wird zunächst entlüftet bzw. benetzt und dann am Katheter bzw. am Griff 26 die Herzpumpe 4 durch die Öffnung 7 aus dem abgeschlossenen ersten Aufnahmeraum 3 heraus- und in ein außerhalb des ersten Aufnahmeraums befindliches Schleusenelement hineingezogen. Dabei wird die Pumpe 4 radial komprimiert. Sie ist danach in dem Schleusenelement 11 sicher und vor Berührung durch den Anwender geschützt gehalten.

Die Pumpe 4 kann vor dem Herausziehen aus dem ersten Aufnahmeraum probeweise betrieben werden, indem von einem im Griffbereich befindlichen Spülsystem aus durch Spülöffnungen eine Spülflüssigkeit über den Katheter 8 zur Pumpe bewegt und diese danach mittels der flexiblen Antriebswelle bei einer gegenüber den Betriebsdrehzahlen deutlich reduzierten Drehzahl betrieben wird.

In Figur 10 ist eine Anordnung, wie sie in Figur 9 als Querschnittsdarstellung gezeigt ist, nochmals in ähnlicher Form in dreidimensionaler Darstellung wiedergegeben.

Mithilfe der Erfindung kann der Behälter 1, 1', 1" (bzw. 11"', siehe Fig. 11b) in der beschriebenen Art ein hohes Maß an Verfügbarkeit und Betriebssicherheit sowie Sterilität der Herzpumpe bei der Implantation gewährleisten.

Fign. 11a und 11b zeigen weitere Ausführungsformen von Aufnahmeräumen. Hierin ist in Fig. 11a zunächst ein Aufnahmeraum gezeigt, bei dem eine Fügestelle 9 in horizontaler Richtung (in Richtung einer Rinne 13 bzw. eines entsprechenden Katheters) angeordnet ist. Zusätzlich ist senkrecht hierzu eine Fügestelle 9" vorgesehen. Diese Fügestellen 9 bzw. 9" können für sich genommen oder auch kumulativ vorgesehen sein. Entsprechend sind Abschlusselemente 5" sowie 6" vorgesehen. Auf der rechten Seite ist (gegenüber der Öffnung 7) eine weitere Öffnung 7a" vorgesehen, deren Durchmesser deutlich größer ist als der Durchmesser der Öffnung 7. Die Größe ist derart bemessen, dass eine Herzpumpe im expandierten bzw. im nur leicht komprimierten Zustand 7a" hineingezogen werden kann. Allerdings kann nach einem Verschluss der Öffnung 7a" die Herzpumpe dann nur noch durch die Öffnung 7 unter Kompression herausgezogen werden. Für diesen Fall ist es möglich, einen Deckel an der Öffnung 7a" vorzusehen, der nicht zerstörungsfrei lösbar ist. Alternativ kann (beispielsweise zur Revision durch den Hersteller) auch eine zerstörungsfreie Abkopplung des Deckels vorgesehen sein. Der Aufnahmeraum 3" bzw. die Auffangwanne 14" sind entsprechend den oben beschriebenen Ausführungsformen zu gestalten. Gleiches gilt für die übrigen Elemente, auf die bereits oben eingegangen wurde (beispielsweise die Rinne 13, ein Katheter 8, eine Herzpumpe 4, etc.).

Fig. 11b zeigt eine weitere Ausführungsform, die sich von der in Fig. 11a dadurch unterscheidet, dass hier lediglich eine Fügestelle 9"' vorgesehen ist. Außerdem ist das unter Abschlusselement (hier mit 5"' bezeichnet) weniger massiv ausgestaltet. Die Auffangwanne 14"' bzw. der Aufnahmeraum 3"' sind prinzipiell wie oben beschrieben, selbiges gilt für die Öffnung 7, die Rinne 13 sowie in den Aufnahmeraum einzubringende Katheter bzw. Herzpumpen. In Fig. 11b ist ein Deckel gezeigt (in Kreuzschraffur), der nicht zerstörungsfrei von den Abschlusselementen 5"', 6'" lösbar ist. In der in Fig. 11b gezeigten Ausführungsform ist die in dem Aufnahmeraum 3'" untergebrachte Herzpumpe 4 beispielhaft gezeigt. Hieraus wird klar, dass die Herzpumpe durch die Öffnung 7a"' in den Aufnahmeraum bringbar ist, allerdings nur unter Kompression durch die Öffnung 7 wieder entnehmbar ist. Die in den Fign. 11a und 11b gezeigten Ausführungsformen können selbstverständlich ohne zweite Öffnung bzw. Deckel vorgesehen sein, in diesem Fall ist die Öffnung 7 die einzige Öffnung der Aufnahmeräume 3" bzw. 3"'. In Fig. 11b ist zumindest angedeutet, dass die Herzpumpe 4 im expandierten Zustand in dem Aufnahmeraum 3"' liegt, und dass dieser expandierte Zustand einen derartigen Durchmesser hat, dass die Pumpe im Wesentlichen kompressionsfrei die Öffnung 7a"' passieren kann, allerdings die Öffnung 7 nur unter Kompression passieren kann.

In einigen Fällen ist es dann möglich, dass das Griffteil 26 erst montier wird, nachdem die Herzpumpe in den Aufnahmeraum 3, 3', 3", 3"' eingebracht wurde. Nach der Montage des Griffes ist die Entnahme der Pumpe nur noch in der vorgesehenen Weise zerstörungsfrei möglich.

Die Anmeldung beinhaltet, unter anderem, die folgenden Aspekte:
1. Behälter (1, 1', 1") für eine Herzpumpeneinrichtung (4, 8, 12, 17, 18, 19, 26) mit einem ersten Aufnahmeraum (3) für eine komprimierbare und expandierbare Herzpumpe (4), wobei der erste Aufnahmeraum (3) auf mehreren Seiten, insbesondere allseitig, von einem oder mehreren Abschlusselementen (5, 5', 6, 6', 6") begrenzt und zur Verhinderung einer Berührung der Herzpumpe nach außen abgeschlossen ist, wobei die Abschlusselemente (5, 5', 6, 6', 6") eine Öffnung (7) zum Durchtritt eines Katheters (8) von außen in den ersten Aufnahmeraum (3) freilassen, wobei der Durchmesser der Öffnung (7) derart bemessen ist, dass die Herzpumpe diese ausschließlich in einem gegenüber dem expandierten Zustand wenigstens teilweise komprimierten Zustand passieren kann.
2. Behälter (1, 1', 1") für eine Herzpumpeneinrichtung (4, 8, 12, 17, 18, 19, 26) mit einem ersten Aufnahmeraum (3) für eine komprimierbare und expandierbare Herzpumpe (4), wobei der erste Aufnahmeraum (3) auf mehreren Seiten, insbesondere allseitig, von einem oder mehreren Abschlusselementen (5, 5', 6, 6', 6") begrenzt und zur Verhinderung einer Berührung der Herzpumpe (4) nach außen abgeschlossen ist, wobei die Abschlusselemente (5, 5', 6, 6', 6") eine Öffnung (7) zum Durchtritt eines Katheters (8) von außen in den ersten Aufnahmeraum (3) freilassen, wobei der Durchmesser der Öffnung kleiner als 6 mm, vorzugsweise kleiner als 5 mm, besonders vorzugsweise kleiner als 4 mm ist.
3. Behälter nach Aspekt 1 oder 2, dadurch gekennzeichnet, dass wenigstens die Abschlusselemente (5, 5', 6, 6', 6"), insbesondere der gesamte Behälter (1, 1', 1"), im Wesentlichen aus einem Kunststoffflachmaterial, insbesondere einer Kunststofffolie, bestehen.
4. Behälter nach Aspekt 1, 2 oder 3, dadurch gekennzeichnet, dass der erste Aufnahmeraum (3) im Wesentlichen durch zwei halbschalenförmige, zusammengefügte Abschlusselemente (5, 5', 6, 6', 6") begrenzt ist.
5. Behälter nach Aspekt 1 oder einem der folgenden, dadurch gekennzeichnet, dass die Abschlusselemente (5, 5', 6, 6', 6") unlösbar miteinander verbunden sind.
6. Behälter nach Aspekt 1 oder einem der folgenden, dadurch gekennzeichnet, dass die Öffnung (7) zum Durchtritt eines Katheters (8) zwischen zwei Abschlusselementen (5, 5', 6, 6', 6") gebildet oder zur Fügestelle (9) zwischen zwei Abschlusselementen hin offen ist.
7. Behälter nach Aspekt 1 oder einem der folgenden, dadurch gekennzeichnet, dass ein erstes der Abschlusselemente (5, 5') als Teil eines Blisters ausgebildet ist, der wenigstens einen Katheter (8), insbesondere zusätzlich weitere Teile einer Herzpumpeneinrichtung aufnimmt.
8. Behälter nach Aspekt 7, dadurch gekennzeichnet, dass das erste Abschlusselement (5, 5') im Bereich des ersten Aufnahmeraums eine Auffangschale (14) für Flüssigkeit bildet.
9. Behälter nach Aspekt 1 oder einem der folgenden, dadurch gekennzeichnet, dass die Öffnung (7) wenigstens teilweise aus einem zylindersymmetrischen Kanal besteht.
10. Behälter nach Aspekt 9, dadurch gekennzeichnet, dass der zylindersymmetrische Kanal sich vom Inneren des ersten Aufnahmeraums (3) aus nach außen verengt.
11. Behälter nach Aspekt 10, dadurch gekennzeichnet, dass die Mündung der Öffnung (7) zur Außenseite der Abschlusselemente (5, 5', 6, 6', 6") einen Rand aufweist, an dem ein längs des Katheters (8) verschiebbares hohlzylindrisches Schleusenelement (11) in Axialrichtung des Kanals abstützbar ist.
12. Behälter nach einem der Aspekte 1 bis 11 mit einer Herzpumpeneinrichtung, wobei eine komprimierbare und expandierbare Herzpumpe (4) sich im ersten Aufnahmeraum (3) befindet und wobei ein mit der Herzpumpe verbundener Katheter (8) durch die Öffnung (7) aus dem ersten Aufnahmeraum (3) herausragt, wobei insbesondere auf dem Katheter frei verschiebbar ein von diesem durchsetztes Schleusenelement (11) vorgesehen ist.
13. Behälter nach einem der vorhergehenden Aspekte, dadurch gekennzeichnet, dass der erste Aufnahmeraum (3) durch mindestens zwei entlang einer Fügelinie gefügte Abschlusselemente begrenzt ist, wobei die Fügelinie im Wesentlichen senkrecht zu einer Passierrichtung der Herzpumpeneinrichtung verläuft.
14. Behälter nach einem der vorhergehenden Aspekte, dadurch gekennzeichnet, dass der erste Aufnahmeraum durch mindestens zwei entlang einer Fügelinie gefügte Anschlusselemente begrenzt ist, wobei die Fügelinie in einem Querschnitt des Aufnahmeraums verläuft, der größer ist als der Querschnitt der Öffnung zum Durchtritt eines Katheters bei der Entnahme der Herzpumpe.
15. Behälter nach einem der vorhergehenden Aspekte, dadurch gekennzeichnet, dass der Aufnahmeraum mehr als eine Öffnung aufweist.
16. Behälter nach Aspekt 15, dadurch gekennzeichnet, dass mindestens eine der Öffnungen im mit der Herzpumpe bestückten Zustand durch einen zerstörungsfrei oder nicht zerstörungsfrei entfernbar gefügten Deckel verschlossen ist.
17. Behälter nach einem der vorhergehenden Aspekte, dadurch gekennzeichnet, dass der Aufnahmeraum so geschaltet ist, dass die Herzpumpeneinrichtung ohne Kompression in den Aufnahmeraum einführbar ist.
18. Verfahren zum Betrieb einer Herzpumpeneinrichtung, bei dem eine Herzpumpe im ersten Aufnahmeraum (3) eines Behälters (1, 1', 1") nach einem der Aspekte 1 bis 17 angeordnet ist und mittels einer durch einen Katheter (8) zur Herzpumpe (4) verlaufenden Welle (12) von außen rotatorisch angetrieben wird.
19. Verfahren nach Aspekt 18, dadurch gekennzeichnet, dass zudem durch eine an dem Katheter (8) vorgesehene Öffnung (18, 19) entlang dem Katheter eine Flüssigkeit zur Herzpumpe (4) gefördert wird.
20. Verfahren zum Betrieb einer Herzpumpeneinrichtung, bei dem eine Herzpumpe (4) im ersten Aufnahmeraum (3) eines Behälters (1, 1', 1") nach einem der Aspekte 1 bis 17 angeordnet ist, wobei die Herzpumpe (4) mit einem Katheter (8) verbunden ist, der durch die Öffnung (7) aus dem ersten Aufnahmeraum (3) herausragt, dadurch gekennzeichnet, dass mittels des Katheters (8) die Herzpumpe (4) durch die Öffnung unter radialer Kompression aus dem ersten Aufnahmeraum (3) heraus und in ein Schleusenelement(11), das auf dem Katheter (8) in Axialrichtung frei verschiebbar ist, hineingezogen wird.

## Patentansprüche

1. Behälter (1, 1', 1") für eine Herzpumpeneinrichtung (4, 8, 12, 17, 18, 19, 26) mit einem ersten Aufnahmeraum (3) für eine komprimierbare und expandierbare Herzpumpe (4), wobei der erste Aufnahmeraum (3) auf mehreren Seiten, insbesondere allseitig, von einem oder mehreren Abschlusselementen (5, 5', 6, 6', 6") begrenzt und zur Verhinderung einer Berührung der Herzpumpe nach außen abgeschlossen ist, wobei die Abschlusselemente (5, 5', 6, 6', 6") eine Öffnung (7) zum Durchtritt eines Katheters (8) von außen in den ersten Aufnahmeraum (3) freilassen, wobei der Durchmesser der Öffnung (7) derart bemessen ist, dass die Herzpumpe diese ausschließlich in einem gegenüber dem expandierten Zustand wenigstens teilweise komprimierten Zustand passieren kann, wobei der erste Aufnahmeraum (3) im Wesentlichen durch zwei halbschalenförmige, zusammengefügte Abschlusselemente (5, 5', 6, 6', 6") begrenzt ist.

2. Behälter (1, 1', 1") nach Anspruch 1, **dadurch gekennzeichnet, dass** der Durchmesser der Öffnung (7) kleiner als 6 mm, vorzugsweise kleiner als 5 mm, besonders vorzugsweise kleiner als 4 mm ist.

3. Behälter nach Anspruch 1 oder 2, **dadurch gekennzeichnet, dass** wenigstens die Abschlusselemente (5, 5', 6, 6', 6"), insbesondere der gesamte Behälter (1, 1', 1"), im Wesentlichen aus einem Kunststoffflachmaterial, insbesondere einer Kunststofffolie, bestehen.

4. Behälter nach Anspruch 1 oder einem der folgenden, **dadurch gekennzeichnet, dass** die Abschlusselemente (5, 5', 6, 6', 6") unlösbar miteinander verbunden sind.

5. Behälter nach Anspruch 1 oder einem der folgenden, **dadurch gekennzeichnet, dass** die Öffnung (7) zum Durchtritt eines Katheters (8) zwischen zwei Abschlusselementen (5, 5', 6, 6', 6") gebildet oder zur Fügestelle (9) zwischen zwei Abschlusselementen hin offen ist, und/oder
**dadurch gekennzeichnet, dass** ein erstes der Abschlusselemente (5, 5') als Teil eines Blisters ausgebildet ist, der wenigstens einen Katheter (8), insbesondere zusätzlich weitere Teile einer Herzpumpeneinrichtung aufnimmt.

6. Behälter nach Anspruch 5, **dadurch gekennzeichnet, dass** das erste Abschlusselement (5, 5') im Bereich des ersten Aufnahmeraums eine Auffangschale (14) für Flüssigkeit bildet.

7. Behälter nach Anspruch 1 oder einem der folgenden, **dadurch gekennzeichnet, dass** die Öffnung (7) wenigstens teilweise aus einem zylindersymmetrischen Kanal besteht.

8. Behälter nach Anspruch 7, **dadurch gekennzeichnet, dass** der zylindersymmetrische Kanal sich vom Inneren des ersten Aufnahmeraums (3) aus nach außen verengt.

9. Behälter nach Anspruch 8, **dadurch gekennzeichnet, dass** die Mündung der Öffnung (7) zur Außenseite der Abschlusselemente (5, 5', 6, 6', 6") einen Rand aufweist, an dem ein längs des Katheters (8) verschiebbares hohlzylindrisches Schleusenelement (11) in Axialrichtung des Kanals abstützbar ist.

10. Behälter nach einem der Ansprüche 1 bis 9 mit einer Herzpumpeneinrichtung, wobei eine komprimierbare und expandierbare Herzpumpe (4) sich im ersten Aufnahmeraum (3) befindet und wobei ein mit der Herzpumpe verbundener Katheter (8) durch die Öffnung (7) aus dem ersten Aufnahmeraum (3) herausragt, wobei insbesondere auf dem Katheter frei verschiebbar ein von diesem durchsetztes Schleusenelement (11) vorgesehen ist, und/oder
**dadurch gekennzeichnet, dass** der erste Aufnahmeraum (3) durch mindestens zwei entlang einer Fügelinie gefügte Abschlusselemente begrenzt ist, wobei die Fügelinie im Wesentlichen senkrecht zu einer Passierrichtung der Herzpumpeneinrichtung verläuft, und/oder
**dadurch gekennzeichnet, dass** der erste Aufnahmeraum durch mindestens zwei entlang einer Fügelinie gefügte Anschlusselemente begrenzt ist, wobei die Fügelinie in einem Querschnitt des Aufnahmeraums verläuft, der größer ist als der Querschnitt der Öffnung zum Durchtritt eines Katheters bei der Entnahme der Herzpumpe, und/oder
**dadurch gekennzeichnet, dass** der Aufnahmeraum mehr als eine Öffnung aufweist.

11. Behälter nach Anspruch 10, **dadurch gekennzeichnet, dass** mindestens eine der Öffnungen im mit der Herzpumpe bestückten Zustand durch einen zerstörungsfrei oder nicht zerstörungsfrei entfernbar gefügten Deckel verschlossen ist.

12. Behälter nach einem der vorhergehenden Ansprüche, **dadurch gekennzeichnet, dass** der Aufnahmeraum so gestaltet ist, dass die Herzpumpeneinrichtung ohne Kompression in den Aufnahmeraum einführbar ist.

13. Verfahren zum Betrieb einer Herzpumpeneinrichtung, bei dem eine Herzpumpe im ersten Aufnahmeraum (3) eines Behälters (1, 1', 1") nach einem der Ansprüche 1 bis 12 angeordnet ist und mittels einer durch einen Katheter (8) zur Herzpumpe (4) verlaufenden Welle (12) von außen rotatorisch angetrieben wird.

14. Verfahren nach Anspruch 13, **dadurch gekennzeichnet, dass** zudem durch eine an dem Katheter (8) vorgesehene Öffnung (18, 19) entlang dem Katheter eine Flüssigkeit zur Herzpumpe (4) gefördert wird.

15. Verfahren zum Betrieb einer Herzpumpeneinrichtung, bei dem eine Herzpumpe (4) im ersten Aufnahmeraum (3) eines Behälters (1, 1', 1") nach einem der Ansprüche 1 bis 12 angeordnet ist, wobei die Herzpumpe (4) mit einem Katheter (8) verbunden ist, der durch die Öffnung (7) aus dem ersten Aufnahmeraum (3) herausragt, **dadurch gekennzeichnet, dass** mittels des Katheters (8) die Herzpumpe (4) durch die Öffnung unter radialer Kompression aus dem ersten Aufnahmeraum (3) heraus und in ein Schleusenelement(11), das auf dem Katheter (8) in Axialrichtung frei verschiebbar ist, hineingezogen wird.

## Claims

1. A container (1, 1', 1") for a heart pump device (4, 8, 12, 17, 18, 19, 26) with a first receiving space (3) for a compressible and expandable heart pump (4), wherein the first receiving space (3) is delimited on several sides, in particular on all sides, by one or more closure elements (5, 5', 6, 6' 6") and is closed off to the outside for preventing a contacting of the heart pump, wherein the closure elements (5, 5', 6, 6' 6") leave free an opening (7) for the passage of a catheter (8) from the outside into the first receiving space (3), wherein the diameter of the opening (7) is dimensioned such that the heart pump can pass this exclusively in a condition which is at least partly compressed compared to the expanded condition, wherein the first receiving space (3) is delimited essentially by two half-shell-shaped, joined-together closure elements (5, 5', 6, 6', 6").

2. A container (1, 1', 1") according to claim 1, **characterised in that** the diameter of the opening (7) is smaller than 6 mm, preferably smaller than 5 mm, particularly preferably smaller than 4 mm.

3. A container according to claim 1 or 2, **characterised in that** at least the closure elements (5, 5', 6, 6', 6"), in particular the complete container (1, 1', 1") consist essentially of a plastic flat material, in particular a plastic foil.

4. A container according to claim 1 or one of the following ones, **characterised in that** the closure elements (5, 5', 6, 6', 6") are undetachably connected to one another.

5. A container according to claim 1 or one of the following ones, **characterised in that** the opening (7) is formed for the passage of a catheter (8) between two closure elements (5, 5', 6, 6', 6") or is open to the joining location (8) between two closure elements, and/or
**characterised in that** a first of the closure elements (5, 5') is designed as part of a blister which receives at least a catheter (8), in particular additionally further parts of a heart pump device.

6. A container according to claim 5, **characterised in that** the first closure element (5, 5') in the region of the first receiving space forms a capture shell (14) for fluid.

7. A container according to claim 1 or one of the following ones, **characterised in that** the opening (7) at least partly consists of a cylinder-symmetrical channel.

8. A container according to claim 7, **characterised in that** the cylinder-symmetrical channel narrows outwardly from the inside of the first receiving space (3).

9. A container according to claim 8, **characterised in that** the run-out of the opening (7) to the outer side of the closure elements (5, 5', 6, 6', 6") comprises an edge, on which a hollow-cylindrical sheath element (11) displaceable along the catheter (8) can be supported in the axial direction of the channel.

10. A container according to one of the claims 1 to 9, with a heart pump device, wherein a compressible and expandable heart pump (4) is located in the first receiving space (3) and wherein a catheter (8) which is connected to the heart pump projects through the opening (7) out of the first receiving space (3), wherein in particular a sheath element (11) through which the catheter passes is provided on the catheter in a freely displaceable manner, and/or
**characterised in that** the first receiving space (3) is delimited by at least two closure elements which are joined along a joining line, wherein the joining line runs essentially perpendicularly to a passing direction of the heart pump device, and/or
**characterised in that** the first receiving space is delimited by at least two closure elements which are joined along a joining line, wherein the joining line runs in a cross section of the receiving space which is larger than the cross section of the opening for the passage of a catheter on removal of the heart pump, and/or
**characterised in that** the receiving space comprises more than one opening.

11. A container according to claim 10, **characterised in that** at least one of the openings in the condition equipped with the heart pump is closed by a cover which is joined in a manner in which it is removable with or without destruction.

12. A container according to one of the preceding claims, **characterised in that** the receiving space is configured such that the heart pump device can be introduced into the receiving space without compression.

13. A method for operating a heart pump device, with which a heart pump is arranged in the first receiving space (3) of a container (1, 1', 1") according to one of the claims 1 to 12 and is externally driven in rotation by way of a shaft (12) running through a catheter (8) to the heart pump (4).

14. A method according to claim 13, **characterised in that** moreover a fluid is delivered to the heart pump (4) along the catheter through an opening (18, 19) provided on the catheter (8).

15. A method for operating a heart pump device, with which a heart pump (4) is arranged in the first receiving space (3) of a container (1, 1', 1") according to one of the claims 1 to 12, wherein the heart pump (4) is connected to a catheter (8) which projects through the opening (7) out of the first receiving space (3), **characterised in that** the heart pump (4), by way of the catheter (8), is pulled through the opening out of the first receiving space (3) amid radial compression and pulled into a sheath element (11) which is freely displaceable on the catheter (8) in the axial direction.

## Revendications

1. Récipient (1, 1', 1") pour un dispositif de pompe cardiaque (4, 8, 12, 17, 18, 19, 26) avec un premier espace de logement (3) pour une pompe cardiaque compressible et dilatable (4), le premier espace de logement (3) étant limité sur plusieurs côtés, plus particulièrement sur tous les côtés, par un ou plusieurs éléments terminaux (5, 5', 6, 6', 6") et fermé afin d'empêcher un contact de la pompe cardiaque avec l'extérieur, les éléments terminaux (5, 5', 6, 6', 6") laissant libre une ouverture (7) pour le passage d'un cathéter (8) de l'extérieur vers le premier espace de logement (3), le diamètre de l'ouverture (7) étant dimensionné de façon à ce que la pompe cardiaque puisse la traverser exclusivement dans un état au moins partiellement comprimé par rapport à l'état dilaté, le premier espace de logement (3) étant essentiellement délimité par deux éléments terminaux (5, 5', 6, 6', 6") assemblés sous la forme de demi-coques.

2. Récipient (1, 1', 1") selon la revendication 1, **caractérisé en ce que** le diamètre de l'ouverture (7) est inférieur à 6 mm, de préférence inférieur à 5 mm, plus particulièrement inférieur à 4 mm.

3. Récipient selon la revendication 1 ou 2, **caractérisé en ce qu'**au moins les éléments terminaux (5, 5', 6, 6', 6"), plus particulièrement l'ensemble du récipient (1, 1', 1"), sont constitués globalement d'une matière plastique plate, plus particulièrement d'un film de matière plastique.

4. Récipient selon la revendication 1 ou l'une des suivantes, **caractérisé en ce que** les éléments terminaux (5, 5', 6, 6', 6") sont reliés entre eux de manière inamovible.

5. Récipient selon la revendication 1 ou l'une des suivantes, **caractérisé en ce que** l'ouverture (7) est conçue pour le passage d'un cathéter (8) entre deux éléments terminaux (5, 5', 6, 6', 6") ou est ouverte en direction d'une jonction (9) entre deux éléments terminaux et/ou **caractérisé en ce qu'**un premier des éléments terminaux (5, 5') est conçu comme une partie d'un blister qui loge au moins un cathéter (8), plus particulièrement en outre d'autres parties d'un dispositif de pompe cardiaque.

6. Récipient selon la revendication 5, **caractérisé en ce que** le premier élément terminal (5, 5') forme, au niveau du premier espace de logement, une coque de réception (14) pour un liquide.

7. Récipient selon la revendication 1 ou l'une des suivantes, **caractérisé en ce que** l'ouverture (7) est constituée au moins partiellement d'un canal à symétrie cylindrique.

8. Récipient selon la revendication 7, **caractérisé en ce que** le canal à symétrie cylindrique se rétrécit de l'intérieur du premier espace de logement (3) vers l'extérieur.

9. Récipient selon la revendication 8, **caractérisé en ce que** l'embouchure de l'ouverture (7) comprend, vers le côté externe des éléments terminaux (5, 5', 6, 6', 6"), un bord contre lequel un élément de sas (11) cylindrique creux et coulissant le long du cathéter (8) peut être appuyé dans la direction axiale du canal.

10. Récipient selon l'une des revendications 1 à 9 avec un dispositif de pompe cardiaque, une pompe cardiaque comprimable et dilatable (4) se trouvant dans le premier espace de logement (3) et un cathéter (8) relié avec la pompe cardiaque dépassant à travers l'ouverture (7) hors du premier espace de logement (3), plus particulièrement un élément de sas (11) coulissant librement sur le cathéter et traversé par celui-ci étant prévu et/ou
**caractérisé en ce que** le premier espace de logement (3) est délimité par au moins deux éléments terminaux assemblés le long d'une ligne de jonction, la ligne de jonction s'étendant globalement perpendiculairement à un dispositif de passage du dispositif de pompe cardiaque et/ou
**caractérisé en ce que** le premier espace de logement est délimité par au moins deux éléments terminaux assemblés le long d'une ligne de jonction, la ligne de jonction s'étendant dans une section transversale de l'espace de logement qui est plus grande que la section transversale de l'ouverture pour le passage d'un cathéter lors du retrait de la pompe cardiaque et/ou
**caractérisé en ce que** l'espace de logement présente plus d'une ouverture.

11. Récipient selon la revendication 10, **caractérisé en ce qu'**au moins une des ouvertures est fermée, dans l'état équipé de la pompe cardiaque, par un couvercle pouvant être retiré sans être détruit ou non.

12. Récipient selon l'une des revendications précédentes, **caractérisé en ce que** l'espace de logement est conçu de façon à ce que le dispositif de pompe cardiaque puisse être introduit sans compression dans l'espace de logement.

13. Procédé d'exploitation d'un dispositif de pompe cardiaque dans lequel une pompe cardiaque est disposée dans le premier espace de logement (3) d'un récipient (1, 1', 1") selon l'une des revendications 1 à 12, et est entraînée en rotation de l'extérieur au moyen d'un arbre (12) s'étendant à travers un cathéter (8) vers la pompe cardiaque (4).

14. Procédé selon la revendication 13, **caractérisé en ce que**, en outre, un liquide est transporté vers la pompe cardiaque (4) le long du cathéter à travers une ouverture (18, 19) prévue sur le cathéter (8).

15. Procédé d'exploitation d'un dispositif de pompe cardiaque, dans lequel une pompe cardiaque (4) est disposée dans le premier espace de logement (3) d'un récipient (1, 1', 1") selon l'une des revendications 1 à 12, la pompe cardiaque (4) étant reliée avec un cathéter (8) qui dépasse à travers l'ouverture (7) hors du premier espace de logement (3), **caractérisé en ce que**, au moyen du cathéter (8), la pompe cardiaque (4) est tirée à travers l'ouverture sous compression radiale hors du premier espace de logement (3) et est tirée dans un élément de sas (11) qui coulisse librement dans la direction axiale sur le cathéter(8).
